# EUROPEAN PATENT SPECIFICATION

(11) **EP 3 285 737 B1**
(45) Date of publication and mention of the grant of the patent: **14.08.2019**
(21) Application number: 16718870.5
(22) Date of filing: 21.04.2016
(51) Int. Cl.: A61K 8/92, A61K 8/39, A61K 8/37, A61K 8/02, A61Q 19/10

(54) **VEGETABLE BUTTER COMPOSITION**
PFLANZENBUTTER ZUSAMMENSETZUNG
COMPOSITION DE BEURRE VÉGÉTAL

(30) Priority: 22.04.2015 GB 201506828
(43) Date of publication of application: 28.02.2018
(73) Proprietor: Cosmetic Warriors Limited, Dorset BH15 1AB (GB)
(72) Inventor: CONSTANTINE, Mark, Poole Dorset BH14 0QD (GB); CONSTANTINE, Margaret Joan, Poole Dorset BH14 0QD (GB); AMBROSEN, Helen Elizabeth, Wimborne Dorset BH21 2NX (GB); BIRD, Rowena Jacqueline, Christchurch, Dorset BH23 4AG (GB)
(74) Representative: D Young & Co LLP
(86) International application number: PCT/GB2016/051100
(87) International publication number: WO 2016/170334

(56) References cited:
- WO-A1-2007/019883
- WO-A2-2007/002431
- WO-A2-2014/068282

## Description

### FIELD OF THE INVENTION

The present invention relates to a solid cosmetic product, a process for producing said product, and a product prepared by the method.

### BACKGROUND TO THE INVENTION

The present invention relates to products particularly those for use in contact with the human body.

Therapeutic bathing products are known in the cosmetic industry and are available in a variety of different forms. Bath salts, oils, and lotions are a few examples of bathing products that can deliver emollients, essential oils and fragrances to a bath, providing calming and remedial properties. However, the required use of solid packaging for these products is a significant disadvantage due to the environmental impact that waste packaging has, despite the availability of recycling.

For at least this reason encapsulated compositions for creating therapeutic bath products have become increasingly popular. For example WO 2014/068282 A2, US 4,294,855 and WO 03/061633 disclose compositions which are capable of enveloping a core cosmetic. The active therapeutic components are often encased in a gelatinous or membrane-bound shell, which dissolves on contact with water, releasing the core ingredients into the water. However the disadvantage of these products is that the shell does not readily dissipate in water, delaying the release of the active ingredients, whilst providing no effective benefit of its own. They are also prone to rupturing, resulting in a wasted and suboptimal product.

The present invention seeks to provide a solid cosmetic product that may be used as a therapeutic bathing product and is capable of delivering a concentrated dose of emollients, essential oils and/or fragrances with an enhanced experience for the user.

### SUMMARY OF THE INVENTION

In a first aspect, there is provided a solid cosmetic composition, as further defined in claim 1, comprising
(i) an inner core comprising
   (a) soft vegetable butter, vegetable oil or a mixture thereof, present in an amount of at least 50 wt.% based on the inner core;
   (b) an emulsifier; and
   (c) a colouring;
(ii) an outer layer comprising
   (a) a hard vegetable butter in an amount of at least 50 wt.% based on the outer layer, and
   (b) a colouring.

In a second aspect, there is provided a process for the production of a solid cosmetic composition comprising an outer layer and an inner core, wherein the outer layer and the inner core are distinct from each other, the process comprising the steps of:
(i) preparing the inner core;
(ii) applying the outer layer to the inner core.

In a third aspect, there is provided a product obtained or obtainable by a process as described herein.

In a fourth aspect, there is provided a cosmetic method comprising contacting the skin of a user with a solid cosmetic composition as described herein.

For ease of reference, these and further aspects of the present invention are now discussed under appropriate section headings. However, the teachings under each section are not necessarily limited to each particular section.

### Advantages

We have identified that by providing a solid cosmetic composition as described herein, a composition is prepared which holds its form and does not readily melt prior to use. By virtue of this property, it is possible to sell the product without unnecessary packaging. We have also found that by providing a solid cosmetic composition comprising an outer layer and an inner core, wherein the outer layer comprises a hard vegetable butter, we are able to provide the user with a product which releases the ingredients contained within the inner core as a result of the outer layer readily dissipating in water.

We have found that vegetable butters act as excellent carriers for oils, colourants and other ingredients, which are desirable for their tranquilising and visual properties, while simultaneously possessing excellent emolliating properties of their own. It was found that it was preferable to use soft vegetable butters for the composition, particularly as the melting point of a soft vegetable butter would allow the composition to readily melt and dissipate. For the avoidance of doubt, there is no predefined bathing temperature range for human use, although it is widely accepted that a comfortable bathing temperature is slightly above human body temperature (on average 37°C) and that an unbearable bathing temperature would be above 47°C.

In addition, we have found that, by including an emulsifier in the inner core, the emulsifier is able to emulsify the fats present in the inner core in order to produce a pleasant visual effect whereby the colouring in the inner core is effectively dispersed throughout the bathing water. It was surprisingly found that, if the emulsifier was included only in the outer layer instead of in the inner core, the dispersal of the colouring from the inner core of the present invention was not achieved as effectively. Furthermore, products in accordance with the present invention provide a distinctive visual droplet effect in use, wherein the components of the outer layer melt to produce coloured droplets of fat which initially float on the surface of the bathing water and do not immediately disperse to combine with the colouring in the inner layer. Furthermore, as the colouring from the outer layer interacts with the emulsifier in the bath water from the inner core, over time, it disperses and mixes with the colouring from the inner layer. If these two colours are different in colour then their mixture will create a new colour in the bath. For example, if the outer layer was coloured blue and the inner layer was coloured yellow, initially yellow bath water with blue fat droplets on the surface would be provided. Over time, the colours would mix to turn the bath water green.

Whilst the use of a soft vegetable butter as a carrier is optimal for use in a bath, we found that there is the potential for the solid composition to deform and become damaged at normal environmental temperatures. This is highly undesirable for transportation and retail purposes, where the finished product must remain stable and maintain a high quality finish. In order to overcome this potential problem, we found that, by coating the core composition with a protective shell comprising hard vegetable butter, we were able to produce a composition that would not deform or become damaged during shipping and on display in a retail environment.

### DETAILED DESCRIPTION

### Composition

As discussed herein, in one aspect of the present invention, there is provided a solid cosmetic composition comprising
(i) an inner core comprising
   (a) soft vegetable butter, vegetable oil or a mixture thereof, as defined herein below or in claim 1, present in an amount of at least 50 wt.% based on the inner core;
   (b) an emulsifier; and
   (c) a colouring;
(ii) an outer layer comprising
   (a) hard vegetable butter ,as defined herein below or in claim 1, in an amount of at least 50 wt.% based on the outer layer, and;
   (b) a colouring.

It will be understood by one skilled in the art that the nature of a cosmetic product means that the product is not edible. Thus in a further aspect the present invention provides a non-edible solid cosmetic composition comprising
(i) an inner core comprising
   (a) soft vegetable butter, vegetable oil or a mixture thereof, as defined herein below or in claim 1, present in an amount of at least 50 wt.% based on the inner core;
   (b) an emulsifier; and
   (c) a colouring;
(ii) an outer layer comprising
   (a) hard vegetable butter ,as defined herein below or in claim 1, in an amount of at least 50 wt.% based on the outer layer, and;
   (b) a colouring.

The solid products of the present invention are compositions which can substantially sustain their physical shape when unsupported by external means, e.g. packaging etc. Thus, they are considered to be solid, solid-like, in solid form or in solid-like form at room temperature. For the avoidance of doubt the solid product must remain substantially solid at up to 30°C.

By solid-like, it is understood that some materials are considered on a day to day basis to be solid, yet over an extremely long period of time, may alter in shape, e.g. amorphous materials such as glass etc. However, they are considered to be solid-like as, for the purpose they fulfil, they are solid.

Due to the solid form of the compositions of the present invention, external packaging is not required to maintain the shape of the composition.

The vegetable butters used in the present invention are triglycerides, which are found to be solid (including solid-like, discussed above) at normal usage temperatures. For the avoidance of doubt the vegetable butter is a triglyceride which remains substantially solid at up to 30°C. It will be appreciated however that it is not a requirement that the vegetable butter have a solid fat content of 100% at normal usage temperatures. In a preferred aspect the solid fat has a solid fat content of at least 70%, preferably at least 80%, preferably at least 90%, preferably at least 95%, preferably at least 98%, preferably at least 99% at 25°C.

The present invention provides a cosmetic product which is solid. The vegetable butters incorporated into the solid product are typically used as agents in bath products. The solid cosmetic composition of the present invention is typically a bath product.

In one aspect the composition of the present invention is made by moulding of the product. In a typical process, the outer layer is formed and placed in a mould. This composition is allowed to solidify to provide a mould shape formed from the outer layer. Into this solidified outer layer is placed on the inner core. If complete enclosure or envelopment of the inner core is required, a further amount of this outer layer is applied to complete the encasement of the inner core.

In another preferred aspect the composition of the present invention is made by preparing the inner core and allowing the core to solidify. The outer layer is prepared by mixing together the components, and warming until all of the component parts have been fully incorporated and melted. The outer layer is subsequently sprayed onto the solid inner core.

In another preferred aspect the composition of the present invention is made by preparing the inner core and allowing the core to solidify. The outer layer is prepared by mixing together the components, and warming until all of the component parts have been fully incorporated and melted. The inner core may then be immersed in the molten mixture of the outer layer such that the inner core is enveloped by the outer layer.

In one preferred aspect the outer layer entirely envelops the inner core.

The term vegetable butter is understood by one skilled in the art and means a triglyceride obtainable from vegetable source which has the consistency of a butter.

### Outer layer

As discussed herein, the outer layer comprises
(a) a hard vegetable butter ,as defined herein below or in claim 1, in an amount of at least 50 wt.% based on the outer layer, and
(b) a colouring.

In one aspect the outer layer is a first vegetable butter composition wherein the first vegetable butter composition comprises
(a) one or more high saturated vegetable butters, wherein the one or more high saturated vegetable butters are selected from vegetable butters having greater than 60wt% saturated fatty acids based on the total fatty acids of the high saturated vegetable butter,
   wherein the total amount of high saturated vegetable butters is at least 50 wt.% based on the first vegetable butter composition, and
(b) a colouring.

The term vegetable butter is understood by one skilled in the art and means a triglyceride obtainable from vegetable source which has the consistency of a butter.

The one or more hard vegetable butters (also known as and referred to as high saturated vegetable butters) is preferably selected from Cocoa butter, Illipe butter, Murumuru butter, Kokum butter and mixtures thereof. In highly preferred aspect, the one or more hard vegetable butters is Cocoa butter.

In a preferred aspect, the outer layer comprises (a) the one or more hard vegetable butters in an amount of at least 50 wt.% based on the outer layer. In a preferred aspect, the outer layer comprises (a) the one or more hard vegetable butters in an amount of at least 60 wt.% based on the outer layer. In a preferred aspect, the outer layer comprises (a) the one or more hard vegetable butters in an amount of at least 70 wt.% based on the outer layer.

In a preferred aspect, the outer layer comprises (a) the one or more hard vegetable butters in an amount of at least 75 wt.% based on the outer layer. In a preferred aspect, the outer layer comprises (a) the one or more hard vegetable butters in an amount of at least 80 wt.% based on the outer layer. In a preferred aspect, the outer layer comprises (a) the one or more hard vegetable butters in an amount of at least 85 wt.% based on the outer layer. In a preferred aspect, the outer layer comprises (a) the one or more hard vegetable butters in an amount of at least 90 wt.% based on the outer layer. In a preferred aspect, the outer layer comprises (a) the one or more hard vegetable butters in an amount of at least 92 wt.% based on the outer layer. In a preferred aspect, the outer layer comprises (a) the one or more hard vegetable butters in an amount of at least 94 wt.% based on the outer layer.

In a preferred aspect, the outer layer comprises (a) the one or more hard vegetable butters in an amount of 50 to 99.99 wt.% based on the outer layer. In a preferred aspect, the outer layer comprises (a) the one or more hard vegetable butters in an amount of 60 to 99.99 wt.% based on the outer layer. In a preferred aspect, the outer layer comprises (a) the one or more hard vegetable butters in an amount of 70 to 99.99 wt.% based on the outer layer. In a preferred aspect, the outer layer comprises (a) the one or more hard vegetable butters in an amount of 75 to 99.99 wt.% based on the outer layer. In a preferred aspect, the outer layer comprises (a) the one or more hard vegetable butters in an amount of 80 to 99.99 wt.% based on the outer layer. In a preferred aspect, the outer layer comprises (a) the one or more hard vegetable butters in an amount of 85 to 99.99 wt.% based on the outer layer. In a preferred aspect, the outer layer comprises (a) the one or more hard vegetable butters in an amount of 90 to 99.99 wt.% based on the outer layer. In a preferred aspect, the outer layer comprises (a) the one or more hard vegetable butters in an amount of 92 to 99.99 wt.% based on the outer layer. In a preferred aspect, the outer layer comprises (a) the one or more hard vegetable butters in an amount of 94 to 99.99 wt.% based on the outer layer.

Thus in one preferred aspect the outer layer comprises (a) a vegetable butter selected from Cocoa butter, Illipe butter, Murumuru butter, Kokum butter and mixtures thereof, in an amount of 80 to 100 wt.% based on the outer layer.

Preferably, the outer layer comprises (b) a colouring in an amount of from 0.001 to 5 wt.% based on the outer layer. In a preferred aspect, the outer layer comprises (b) a colouring in an amount of from 0.001 to 4 wt.% based on the outer layer. In a preferred aspect, the outer layer comprises (b) a colouring in an amount of from 0.001 to 3 wt.% based on the outer layer. In a preferred aspect, the outer layer comprises (b) a colouring in an amount of from 0.001 to 2 wt.% based on the outer layer. In a preferred aspect, the outer layer comprises (b) a colouring in an amount of from 0.001 to 1 wt.% based on the outer layer. In a preferred aspect, the outer layer comprises (b) a colouring in an amount of from 0.01 to 1 wt.% based on the outer layer. In a preferred aspect, the outer layer comprises (b) a colouring in an amount of from 0.1 to 1 wt.% based on the outer layer.

In one aspect, the colouring is selected from an oil soluble colouring, a water soluble colouring, and mixtures thereof. In a preferred aspect, the colouring is a water soluble colouring. In a preferred aspect, the colouring is an oil soluble colouring. The colouring can be naturally derived or it can be a synthetic colouring.

The essential components of the outer layer are defined herein. However the composition may contain further additional components. For example, the outer layer may contain one or more additional vegetable butters that are not specified herein.

In one aspect, the outer layer further comprises a soft vegetable butter. Preferably, the soft vegetable butter may be selected from Aloe butter, Avocado butter, Cupuacu butter, Macadamia Nut butter, Mango butter, Olive butter, Shea butter, Coconut butter, Pumpkin Seed butter, Peanut butter, Almond butter, Coffee Bean butter, Refined butter, Hemp Seed butter, Mochacchino butter, Pistachio Nut butter, Shealoe butter and mixtures thereof.

Preferably the outer layer comprises the soft vegetable butter in an amount of from 0.001 to 30 wt.% based on the outer layer. Preferably the outer layer comprises the soft vegetable butter in an amount of from 0.001 to 20 wt.% based on the outer layer. Preferably the outer layer comprises the soft vegetable butter in an amount of from 0.001 to 10 wt.% based on the outer layer. Thus in a preferred aspect, the present invention provides a solid cosmetic composition comprising
(i) an inner core comprising
   (a) soft vegetable butter, vegetable oil or a mixture thereof, as defined herein below or in claim 1, present in an amount of at least 50 wt.% based on the inner core;
   (b) an emulsifier; and
   (c) a colouring;
(ii) an outer layer comprising
   (a) a hard vegetable butter ,as defined herein below or in claim 1, in an amount of from 80 to 99.99 wt.% based on the outer layer;
   (b) a colouring in an amount of from 0.001 to 5 wt.% based on the outer layer; and
   (c) soft vegetable butter in an amount of from 0 to 10 wt.% based on the outer layer.

In one aspect, the outer layer further comprises an oil, such as a vegetable, nut or plant oil. Preferably the outer layer comprises the oil in an amount of 0.001 to 10 wt.% based on the outer layer. Preferably, the outer layer comprises the oil in an amount of 0.001 to 5 wt.% based on the outer layer. Thus in a preferred aspect, the present invention provides a solid cosmetic composition comprising
(i) an inner core comprising
   (a) soft vegetable butter, vegetable oil or a mixture thereof, as defined herein below or in claim 1, present in an amount of at least 50 wt.% based on the inner core;
   (b) an emulsifier; and
   (c) a colouring;
(ii) an outer layer comprising
   (a) a hard vegetable butter ,as defined herein below or in claim 1, in an amount of from 80 to 99.99 wt.% based on the outer layer, and
   (b) a colouring in an amount of from 0.001 to 5 wt.% based on the outer layer; and
   (c) an oil selected from vegetable oils, nut oils and plant oils, wherein the oil is present in an amount of 0.001 to 5 wt.% based on the outer layer.

In one aspect the outer layer further comprises an emulsifier. Preferably the emulsifier is present in an amount of from 0.001 to 10 wt.% based on the outer layer. Preferably the emulsifier is present in an amount of from 0.001 to 5 wt.% based on the outer layer. Preferably the emulsifier is selected from polyoxyether of lauryl alcohol, PEG-6 caprylic/capric glycerides, PEG-60 almond glycerides, sodium cocoamphoacetate, polyglycerol-3-stearate and mixtures thereof. Preferably the emulsifier is a mixture of PEG-6 caprylic/capric glycerides and PEG-60 almond glycerides. Preferably the emulsifier is a mixture of PEG-6 caprylic, capric glycerides and PEG-60 almond glycerides.

In one aspect the outer layer further comprises an effervescent material. The effervescent material may be selected from appropriate material which effervesces on contact with water. Inclusion of the effervescent material in the outer layer provides an improved visual effect for the user, and also enables the dispersion of the colouring in the outer layer to be achieved more rapidly.

In one aspect the effervescent material comprises at least sodium bicarbonate and citric acid. These materials are present in any suitable amounts to achieve effervescence. One skilled in the art is able to combine these materials to provide the desired rate of effervescence.

### Inner core

As discussed herein, the inner core comprises
(a) soft vegetable butter, vegetable oil or a mixture as defined herein below or in claim 1, present in an amount of at least 50 wt.% based on the inner core;
(b) an emulsifier; and
(c) a colouring.

In one aspect the inner core is a second vegetable butter composition wherein the second vegetable butter composition comprises
(a) one or more low saturated vegetable butters, wherein the one or more low saturated vegetable butters are selected from vegetable butters having less than 60wt% saturated fatty acids based on the total fatty acids of the low saturated vegetable butter,
   wherein the total amount of low saturated vegetable butters is at least 50 wt.% based on the second vegetable butter composition;
(b) an emulsifier; and
(c) a colouring.

Preferably the one or more soft vegetable butters of the inner core is selected from Aloe butter, Avocado butter, Cupuacu butter, Macadamia Nut butter, Mango butter, Olive butter, Shea butter, Coconut butter, Pumpkin Seed butter, Peanut butter, Almond butter, Coffee Bean butter, Refined butter, Hemp Seed butter, Mochacchino butter, Pistachio Nut butter, Shealoe butter and mixtures thereof.

In a preferred aspect, the inner core comprises (a) the one or more soft vegetable butters in an amount of at least 50 wt.% based on the inner core. In a preferred aspect, the inner core comprises (a) the one or more soft vegetable butters in an amount of at least 55 wt.% based on the inner core. In a preferred aspect, the inner core comprises (a) the one or more soft vegetable butters in an amount of at least 60 wt.% based on the inner core. In a preferred aspect, the inner core comprises (a) the one or more soft vegetable butters in an amount of at least 62 wt.% based on the inner core. In a preferred aspect, the inner core comprises (a) the one or more soft vegetable butters in an amount of at least 64 wt.% based on the inner core. In a preferred aspect, the inner core comprises (a) the one or more soft vegetable butters in an amount of at least 66 wt.% based on the inner core. In a preferred aspect, the inner core comprises (a) the one or more soft vegetable butters in an amount of at least 68 wt.% based on the inner core.

In one preferred aspect the one or more soft vegetable butters is present in an amount of 50 to 99.99 wt.% based on the inner core, such as in an amount of 55 to 99.99 wt.% based on the inner core, such as in an amount of 60 to 99.99 wt.% based on the inner core, such as in an amount of 62 to 99.99 wt.% based on the inner core, such as in an amount of 64 to 99.99 wt.% based on the inner core, such as in an amount of 66 to 99.99 wt.% based on the inner core, such as in an amount of 68 to 99.99 wt.% based on the inner core.

In one preferred aspect, the one or more soft vegetable butters is present in an amount of 50 to 90 wt.% based on the inner core, such as in an amount of 50 to 85 wt.% based on the inner core, such as in an amount of 50 to 80 wt.% based on the inner core, such as in an amount of 55 to 80 wt.% based on the inner core, such as in an amount of 60 to 80 wt.% based on the inner core, such as in an amount of 65 to 80 wt.% based on the inner core.

In one aspect the inner core comprises
(a) a vegetable oil;
(b) an emulsifier; and
(c) a colouring.

In a preferred aspect, the inner core comprises
(a) a mixture of soft vegetable butter and vegetable oil in an amount of at least 50 wt.% based on the inner core;
(b) an emulsifier; and
(c) a colouring.

Preferably, the vegetable oil is present in an amount of from 0.001 to 20 wt.% based on the inner core, such as in an amount of 0.001 to 15 wt.% based on the inner core, such as in an amount of from 0.01 to 15 wt.% based on the inner core, such as in an amount of from 0.1 to 15 wt.% based on the inner core, such as in an amount of from 5 to 15 wt.% based on the inner core, such as in an amount of from 5 to 10 wt.% based on the inner core, such as in an amount of from 10 to 15 wt.% based on the inner core, such as in an amount of from 8 to 12 wt.% based on the inner core.

Preferably, the vegetable oil is selected from almond oil, jojoba oil, castor oil, olive oil, grape seed oil, argan oil, moringa oil, baobab oil, rose hip oil, kalahari melon oil, brazil nut oil and mixtures thereof.

In one aspect the inner core comprises (b) an emulsifier in an amount of from 3 to 20 wt.% based on the inner core, such as in an amount of from 5 to 15 wt.% based on the inner core, such as in an amount of from 5 to 12 wt.% based on the inner core, such as in an amount of from 8 to 12 wt.% based on the inner core.

Preferably the emulsifier is selected from polyoxyether of lauryl alcohol, PEG-6 caprylic/capric glycerides, PEG-60 almond glycerides, sodium cocoamphoacetate, polyglycerol-3-stearate and mixtures thereof.

In a preferred aspect the inner core comprises a colouring in an amount of from 0.001 to 5 wt.% based on the inner core. In a preferred aspect, the inner core comprises (c) a colouring in an amount of from 0.001 to 4 wt.% based on the inner core. In a preferred aspect, the inner core comprises (c) a colouring in an amount of from 0.001 to 3 wt.% based on the inner core. In a preferred aspect, the inner core comprises (c) a colouring in an amount of from 0.001 to 2 wt.% based on the inner core. In a preferred aspect, the inner core comprises (c) a colouring in an amount of from 0.001 to 1 wt.% based on the inner core. In a preferred aspect, the inner core comprises (c) a colouring in an amount of from 0.01 to 1 wt.% based on the inner core. In a preferred aspect, the inner core comprises (c) a colouring in an amount of from 0.1 to 1 wt.% based on the inner core.

In one aspect, the colouring is selected from an oil soluble colouring, a water soluble colouring, and mixtures thereof. In a preferred aspect, the colouring is an oil soluble colouring. In a preferred aspect the colouring is a water soluble colouring. The colouring can be naturally derived or it can be synthetic.

The essential components of the inner core are defined herein. However the composition may contain further additional components. For example, the soft vegetable butter composition may contain one or more additional vegetable butters not specified herein.

In one aspect the inner core further comprises a hard vegetable butter. Preferably the hard vegetable butter is selected from Cocoa butter, Illipe butter, Murumuru butter, Kokum butter and mixtures thereof.

Preferably the inner core comprises the hard vegetable butter in an amount of from 1 to 40 wt.% based on the inner core. Preferably the inner core comprises the hard vegetable butter in an amount of from 1 to 35 wt.% based on the inner core. Preferably the inner core comprises the hard vegetable butter in an amount of from 1 to 30 wt.% based on the inner core. Preferably the inner core comprises the hard vegetable butter in an amount of from 1 to 25 wt.% based on the inner core. Preferably the inner core comprises the hard vegetable butter in an amount of from 5 to 25 wt.% based on the inner core. Preferably the inner core comprises the hard vegetable butter in an amount of from 10 to 25 wt.% based on the inner core. Preferably the inner core comprises the hard vegetable butter in an amount of from 10 to 20 wt.% based on the inner core. Preferably the inner core comprises the hard vegetable butter in an amount of from 15 to 25 wt.% based on the inner core. Preferably the inner core comprises the hard vegetable butter in an amount of from 15 to 20 wt.% based on the inner core.

In one aspect the inner core further comprises an effervescent material. The effervescent material may be selected from appropriate material which effervesces on contact with water. Inclusion of the effervescent material in the inner provides an improved visual effect for the user, and also enables the dispersion of the colouring from within the inner core to be achieved more rapidly.

In one aspect the effervescent material comprises at least sodium bicarbonate and citric acid. These materials are present in any suitable amounts to achieve effervescence. One skilled in the art is able to combine these materials to provide the desired rate of effervescence.

### Outer layer & Inner core

The outer and inner layer may be present in suitable relevant amounts to provide the desired properties for the product. In one aspect, the outer layer is 1 to 30 wt.% and the inner core is 70-99 wt.%, based on the combined weight of the inner core and outer layer. In one aspect, the outer layer is 1 to 25 wt.% and the inner core is 75-99 wt.%, based on the combined weight of the inner core and outer layer. In one aspect, the outer layer is 1 to 20 wt.% and the inner core is 80-99 wt.%, based on the combined weight of the inner core and outer layer. In one aspect, the outer layer is 1 to 15 wt.% and the inner core is 85-99 wt.%, based on the combined weight of the inner core and outer layer. In one aspect, the outer layer is 1 to 10 wt.% and the inner core is 90-99 wt.%, based on the combined weight of the inner core and outer layer. In one aspect, the outer layer is 5 to 10 wt.% and the inner core is 90-95 wt.%, based on the combined weight of the inner core and outer layer.

The combined amount of the vegetable butters may be present in any amount to provide the desired physical characteristics of the solid cosmetic product. Preferably vegetable butter is present in the solid cosmetic product in an amount of from about 10% to about 99% by weight of the total composition. Preferably vegetable butter is present in the solid cosmetic product in an amount of from about 20% to about 99% by weight of the total composition. Preferably vegetable butter is present in the solid cosmetic product in an amount of from about 30% to about 99% by weight of the total composition. Preferably vegetable butter is present in the solid cosmetic product in an amount of from about 40% to about 99% by weight of the total composition. Preferably vegetable butter is present in the solid cosmetic product in an amount of from about 50% to about 99% by weight of the total composition. Preferably vegetable butter is present in the solid cosmetic product in an amount of from about 60% to about 99% by weight of the total composition. Preferably vegetable butter is present in the solid cosmetic product in an amount of from about 70% to about 99% by weight of the total composition.

The combined amount of the outer layer and the inner core in any amount to provide the desired physical characteristics of the solid cosmetic product. Preferably the combined amount of the outer layer and the inner core is from about 10% to about 100% by weight of the total composition. Preferably the combined amount of the outer layer and the inner core is from about 20% to about 100% by weight of the total composition. Preferably the combined amount of the outer layer and the inner core is from about 30% to about 100% by weight of the total composition. Preferably the combined amount of the outer layer and the inner core is from about 40% to about 100% by weight of the total composition. Preferably the combined amount of the outer layer and the inner core is from about 50% to about 100% by weight of the total composition. Preferably the combined amount of the outer layer and the inner core is from about 60% to about 100% by weight of the total composition. Preferably the combined amount of the outer layer and the inner core is from about 70% to about 100% by weight of the total composition. Preferably the combined amount of the outer layer and the inner core is from about 80% to about 100% by weight of the total composition. Preferably the combined amount of the outer layer and the inner core is from about 85% to about 100% by weight of the total composition. Preferably the combined amount of the outer layer and the inner core is from about 90% to about 100% by weight of the total composition. Preferably the combined amount of the outer layer and the inner core is from about 95% to about 100% by weight of the total composition. Preferably the combined amount of the outer layer and the inner core is 100% by weight of the total composition.

In some embodiments, the outer layer comprises an oil soluble colouring and the inner core comprises a water soluble colouring. In some embodiments, the outer layer comprises a water soluble colouring and the inner core comprises an oil soluble colouring. In some embodiments, both the outer layer and the inner core comprise a water soluble colouring. In some embodiments, both the outer layer and the inner core comprise an oil soluble colouring. In some embodiments, the outer layer comprises a mixture of an oil soluble colouring and a water soluble colouring, and the inner core comprises a water soluble colouring. In particular, an advantageous effect is achieved by combining oil soluble and water soluble colourings in the outer layer. In this embodiment the bathing water can be coloured by a water soluble colouring present in the inner core. When the outer layer melts in the bathing water, the oil soluble and water soluble colourings are initially dispersed to produce the above-noted droplet effect. After a period of time, a colour change is observed in the water. This is due to the blending of two separate water colourings sourced from the inner core and the outer layer.

### Additional Components

The solid product of the present invention may also comprise one or more cosmetically acceptable additives. The person skilled in the art is aware of a range of cosmetically acceptable additives which are suitable for incorporation into such compositions. For example, binders, fillers, opacifiers, perfumes, fragrances, decorative items and mixtures thereof.

It is particularly preferred that the composition of the present invention further comprises a fragrance. Preferably the fragrance is selected from essential oils. Preferably the fragrance, and more preferably the essential oil, is present in the inner core in an amount of from 0.001 to 10 wt.% of the inner core, and/or is present in the outer layer in an amount of from 0.001 to 10 wt.% based on the outer layer. Preferably the fragrance, and more preferably the essential oil, is present in the inner core in an amount of from 0.001 to 8 wt.% of the inner core, and/or is present in the outer layer in an amount of from 0.001 to 8 wt.% based on the outer layer. Preferably the fragrance, and more preferably the essential oil, is present in the inner core in an amount of from 0.001 to 6 wt.% of the inner core, and/or is present in the outer layer in an amount of from 0.001 to 6 wt.% based on the outer layer. Preferably the fragrance, and more preferably the essential oil, is present in the inner core in an amount of from 1 to 6 wt.% of the inner core, and/or is present in the outer layer in an amount of from 1 to 6 wt.% based on the outer layer. Preferably the fragrance, and more preferably the essential oil, is present in the inner core in an amount of from 4 to about 6 wt.% of the inner core, and/or is present in the outer layer in an amount of from 4 to 6 wt.% based on the outer layer. Preferably the fragrance, and more preferably the essential oil, is present in the inner core in an amount of approximately 5 wt.% of the inner core, and/or is present in the outer layer in an amount of approximately 5 wt.% based on the outer layer.

In one aspect the outer layer comprises a fragrance in an amount of 1 to 10 wt.% based on the outer layer, and wherein the inner core comprises a fragrance in an amount of 1 to 10 wt.% based on the inner core.

Fruit and herb extracts and juices, vegetable oils and essential oils are all compatible with the composition.

In one embodiment, the cosmetically acceptable additives are selected from the group consisting of essential oils, vitamins, fragrances, colourings, clays, decorative articles, and mixtures thereof.

The essential oils may be selected based on the fragrance desired, skin type to be treated and other effects desired based on the well known properties of essential oils. The addition of essential oils, when taken in to the nose, are known to alter mood. For example, essential oils are known to create effects of drowsiness or stimulating the senses. Many well documented effects can be achieved by the use of essential oils.

In one embodiment, the one or more essential oils present in the solid product are selected from Tarragon, Lemon myrtle, Jasmin, Ylang ylang, Labdunum, Lemongrass, Rose otto, Grapefruit, Patchouli, Rosemary, Armois, Lemon, Neroli, Sweet violet, Lavender, Orange 50 fold, Vanilla, Peppermint, Benzoin, Hydrangia, Litsea Cubeba, Cardamon, Tonka, and Chamomile blue. In one embodiment, the one or more essential oils present in the solid product are selected from Tarragon, Lemon myrtle, Labdunum, and Lemon.

Vitamins, particularly B, C and E are very beneficial for the skin. Vitamin rich ingredients such as Wheatgerm oil can also be used to deliver vitamins on to the skin. In a one embodiment, the vitamins are selected from vitamin B, vitamin C, vitamin E and mixtures thereof. It will be appreciated by one skilled in the art that the vitamin may be provided from any suitable source. For example the vitamin(s) may be provided from a synthetic source or from incorporation into the solid product of a material, such as a natural material, that has a high vitamin content.

The ingredients in the present invention do not require cosmetic preservatives. The use of cosmetic preservatives can increase the potential to irritate the skin.

The decorative items which may be present in the solid product include items such as glitter, paper such as rice paper, sequins, dried or fresh flowers, herbs, vegetables, parts thereof or mixtures thereof. Other enhancing materials may also be incorporated
Further preferred additive materials include vegetable oils, chocolate, herbs and spices, cosmetic colours (e.g. paprika, gardenia extract, D&C red no. 30), beans (e.g. aduki), fruit, fresh or dried (e.g. banana, avocado, mango, papaya, kiwi, raspberry, strawberry, blueberries, grapes, tomato, asparagus, or cucumber), honey, glycerin, cosmetic glitter, other vegetable butters (e.g. mango, avocado), clays (e.g. kaolin), starches (e.g. corn starch), popping candy and mixtures thereof.

The above ranges provide preferred amounts of each of the components. Each of these ranges may be taken alone or combined with one or more other component ranges to provide a preferred aspect of the invention.

### Process

As discussed herein, the invention provides a process for the production of a solid composition as described herein;
the process comprising the steps of:
i) preparing the inner core;
ii) applying the outer layer to the inner core.

The shape of the solid products of the present invention is not limited. It may be that the solid products are provided with a shape which would be aesthetically pleasing and/ or which aids in the use of the product. For example, it may be that the solid product is produced in such a manner so that it solidifies in a shape which is ergonomically acceptable to the user. Products which are approximately spherical, cuboidal or cylindrical are each envisaged. Therefore, in one embodiment of the process of the present invention, the mixture of step i) and/or step ii) is pressed into a mould, allowed to solidify, and then turned out to produce the solid product.

In one aspect, when the mixture of step i) and/or step ii) is pressed into a mould, allowed to solidify and then turned out to produce the solid product, the outer layer is tempered. The skilled person understands that the term tempered refers to a product which has undergone heat treatment in order to increase the shine and durability of a coating. It is well known that uncontrolled crystallisation of hard vegetable butters typically results in crystals of varying size resulting in a mottling of the surface and a dull appearance. Tempering the outer layer of the product of the present invention results in a coating which has an improved appearance and durability.

Tempering is achieved in the present invention by initially cooling the resultant product, reheating the product, and then cooling once more to achieve the finished solid cosmetic composition.

In one embodiment, the inner core is prepared by pouring a molten composition into a mould and allowed to solidify.

In a further embodiment, the inner core is moulded by hand and allowed to solidify.

In one embodiment, the outer layer is applied to the inner core by
(i) preparing the outer layer composition;
(ii) heating the outer layer composition to a temperature at which it is liquid;
(iii) applying the outer layer to the inner core by spraying.

In a further embodiment, the outer layer is applied to the inner core by
(i) preparing the outer layer composition;
(ii) heating the outer layer composition to a temperature at which it is liquid;
(iii) applying the outer layer to the inner core by immersing the inner core in the molten outer layer.

In a further embodiment, the outer layer is applied to the inner core by
(i) preparing the outer layer composition;
(ii) placing the outer layer in a mould and allowing to solidify;
(iii) placing the inner core within the solidified outer layer in the mould;
(iv) if complete enclosure or envelopment of the inner core is required, applying a further amount of the outer layer to complete the encasement of the inner core.

As described herein, the solid product may further comprise one or more cosmetically acceptable additives. In one embodiment, the process further comprises the step of combining with the mixture of step i) and/or step ii) one or more cosmetically acceptable additives as defined herein and/or the dispersant defined herein.

The present invention also provides a product obtained or obtainable by a process as described herein.

### Method

In one aspect of the present invention, there is provided a method comprising contacting the skin of a user with water in which the solid cosmetic composition as defined herein has been placed in, or has dissolved in, or in which the solid cosmetic composition as defined herein is dissolving. In a typical method, water is run in to the bath at an acceptable temperature. The user immerses their body in the water and the solid cosmetic composition is dropped into the water. The user then watches the effect of the product on the surface of the water as the outer layer dissolves and disperses its colouring, whilst releasing the components of the inner core. The colouring of the inner core is thus dispersed through the water. The user then bathes in the water.

### Example

The invention will now be described with reference to the following non-limiting example.

A general methodology for preparing compositions in accordance with the present invention is as follows:
1. Warm the hard vegetable butter of phase A to between 50°C - 90°C until it has completely melted.
2. Add the remaining materials of phase A to the molten mix and blend together at a temperature of between 50°C - 80°C until all of the component parts have been fully incorporated.
3. Incorporate phase B into the phase A mixture at a temperature of between 40°C - 55°C.
4. Cool the mixture to a temperature of between 20°C - 40°C and add phases C and D to the overall mix and blend together.
5. Pour the mixture into moulds and cool the mixture to between 4°C - 20°C to produce the inner core.
6. Warm the hard vegetable butter of phase E to between 50°C - 95°C until it has completely melted.
7. Add the remaining materials of phase E to the molten mix and blend together at a temperature of between 50°C - 80°C until all of the component parts have been fully incorporated.
8. Add the materials of phase F to the phase E mixture at a temperature of between 40°C - 55°C.
9. Cool the mixture to 40°C and apply a fine layer of the outer mix composition to the inner core.
10. Cool the resulting composition to between 4°C - 30°C and apply decorative coating materials of phase G.

A solid cosmetic composition having the following composition was prepared. The amounts of each component are provided as wt.% based on the inner core or outer layer or coating, depending on which layer the component is comprised in.

| **Batch Size:** | | | **200.00g** |
|---|---|---|---|
| **Phase** | **Formula wt.%** | **Raw Material Type** | |
| **INNER CORE** | | | |
| | wt.% (based on component) | | wt. (g) |
| **A** | 10 | Murumuru Butter | 18 |
| | 67.9 | Cupuacu Butter | 122.22 |
| | 10 | Castor Oil | 18 |
| | 2 | Castor Sugar | 3.6 |
| | 2 | Cornflour | 3.6 |
| | | | |
| **B** | 6 | PEG-6 caprylic/capric glycerides & PEG-60 almond glycerides | 10.8 |
| | | | |
| **C** | 0.1 | Crocin Extract | 0.18 |
| | | | |
| **D** | 2 | Fragrance | 3.6 |
| | | | |
| | **100%** | | |
| | | | |

| **OUTER LAYER** | | | |
|---|---|---|---|
| | wt.% (based on component) | | wt. (g) |
| **E** | 93.995 | Illipe Butter | 17.859 |
| | 2 | Avocado Butter | 0.38 |
| | 1 | Olive Oil | 0.19 |
| | | | |
| **F** | 1 | Dried Rose Petals | 0.19 |
| | 0.005 | Chlorophyllin Powder | 0.001 |
| | 2 | Fragrance | 0.38 |
| | | | |
| | **100%** | | |
| | | | |

| **COATING** | | | |
|---|---|---|---|
| | wt. % (based on component) | | wt. (g) |
| **G** | 50 | Dried Rose Petals | 0.5 |
| | 50 | Green Glimmer Lustre | 0.5 |
| | | | |
| | **100%** | | **200** |

It was found that the prepared samples could be handled without undue oiling of the outer layer.

## Claims

1. A solid cosmetic composition comprising
(i) an inner core which is a second vegetable butter composition wherein the second vegetable butter composition comprises
(a) one or more low saturated vegetable butters, wherein the one or more low saturated vegetable butters are selected from vegetable butters having less than 60wt% saturated fatty acids based on the total fatty acids of the low saturated vegetable butter, wherein the total amount of low saturated vegetable butters is at least 50 wt.% based on the second vegetable butter composition;
(b) an emulsifier; and
(c) a colouring;
(ii) an outer layer which is a first vegetable butter composition wherein the first vegetable butter composition comprises
(a) one or more high saturated vegetable butters, wherein the one or more high saturated vegetable butters are selected from vegetable butters having greater than 60wt% saturated fatty acids based on the total fatty acids of the high saturated vegetable butter, wherein the total amount of high saturated vegetable butters is at least 50 wt.% based on the first vegetable butter composition, , and
(b) a colouring.

2. A solid cosmetic composition according to claim 1 wherein the inner core is 90-99 wt.% and the outer layer is 1 to 10 wt.%, based on the combined weight of the inner core and outer layer.

3. A solid cosmetic composition according to claim 1 or 2 wherein the inner core comprises low saturated vegetable butters in an amount of from 50 to 80 wt.% based on the inner core.

4. A solid cosmetic composition according to claim 1, 2 or 3 wherein the inner core comprises emulsifier in an amount of from 3 to 20 wt.% based on the inner core; and/orwherein the emulsifier is selected from polyoxyether of lauryl alcohol, PEG-6 caprylic/capric glycerides, PEG-60 almond glycerides, sodium cocoamphoacetate and mixtures thereof.

5. A solid cosmetic composition according to any one of claims 1 to 4 wherein the inner core comprises colouring in an amount of from 0.001 to 5 wt.% based on the inner core; and/or wherein the colouring in the inner core is a water soluble colouring.

6. A solid cosmetic composition according to any one of claims 1 to 5 wherein the inner core further comprises high saturated vegetable butters ; such as the inner core comprises high saturated vegetable butters in an amount of from 5 to 25 wt.% based on the inner core.

7. A solid cosmetic composition according to any one of claims 1 to 6 wherein the outer layer comprises high saturated vegetable butters in an amount of at least 60 wt.% based on the outer layer; such as the outer layer comprises hard vegetable butter in an amount of from 80 to 99.999 wt.% based on the outer layer.

8. A solid cosmetic composition according to any one of claims 1 to 7 wherein the outer layer comprises colouring in an amount of from 0.001 to 5 wt.% based on the outer layer; and/or wherein the colouring in the outer layer is an oil soluble colouring.

9. A solid cosmetic composition according to any one of claims 1 to 8 wherein the outer layer further comprises low saturated vegetable butters ; such as the outer layer comprises low saturated vegetable butters in an amount of from 0.001 to 10 wt.% based on the outer layer.

10. A solid cosmetic composition according to any one of claims 1 to 9 wherein the outer layer further comprises an emulsifier.

11. A solid cosmetic composition according to claim 1 comprising
(i) an inner core comprising
(a) low saturated vegetable butters in an amount of from 50 to 80 wt.% based on the inner core;
(b) an emulsifier in an amount of from 3 to 20 wt.% based on the inner core; and
(c) a colouring in an amount of from 0.001 to 5 wt.% based on the inner core;
(ii) an outer layer comprising
(a) high saturated vegetable butters in an amount of from 80 to 99.999 wt.% based on the outer layer,
(b) a colouring in an amount of from 0.001 to 5 wt.% based on the outer layer; and
(c) soft vegetable butter in an amount of from 0 to 10 wt.% based on the outer layer.

12. A solid cosmetic composition according to any one of claims 1 to 11 wherein the high saturated vegetable butters are selected from Cocoa butter, Illipe butter, Murumuru butter, Kokum butter and mixtures thereof.

13. A solid cosmetic composition according to any one of claims 1 to 12, wherein the low saturated vegetable butters are selected from Aloe butter, Avocado butter, Cupuacu butter, Macadamia Nut butter, Mango butter, Olive butter, Shea butter, Coconut butter, Pumpkin Seed butter, Peanut butter, Almond butter, Coffee Bean butter, Refined butter, Hemp Seed butter, Mochacchino butter, Pistachio Nut butter, Shealoe butter and mixtures thereof.

14. A process for the production of a solid cosmetic composition as defined in claims 1 to 13 comprising the steps of:
i) preparing the inner core;
ii) applying the outer layer to the inner core.

15. A process according to claim 14 wherein the outer layer is applied to the inner core by
(a) preparing the outer layer composition
(b) heating the outer layer composition to a temperature at which it is liquid
(c) applying the outer layer to the inner core by spraying.

## Patentansprüche

1. Feste kosmetische Zusammensetzung, umfassend
(i) einen inneren Kern, der eine zweite pflanzliche Butterzusammensetzung ist, wobei die zweite pflanzliche Butterzusammensetzung umfasst:
(a) eine oder mehrere pflanzliche Butterarten mit wenig Sättigung, wobei die eine oder mehreren pflanzlichen Butterarten mit wenig Sättigung ausgewählt sind aus pflanzlichen Butterarten mit weniger als 60 Gew.-% an gesättigten Fettsäuren, bezogen auf die gesamten Fettsäuren der pflanzlichen Butter mit wenig Sättigung, wobei die Gesamtmenge an pflanzlichen Butterarten mit wenig Sättigung wenigstens 50 Gew.-%, bezogen auf die zweite pflanzliche Butterzusammensetzung, beträgt;
(b) einen Emulgator; und
(c) einen Farbstoff;
(ii) eine äußere Schicht, die eine erste pflanzliche Butterzusammensetzung ist, wobei die erste pflanzliche Butterzusammensetzung umfasst:
(a) eine oder mehrere pflanzliche Butterarten mit hoher Sättigung, wobei die eine oder mehreren pflanzlichen Butterarten mit hoher Sättigung ausgewählt sind aus pflanzlichen Butterarten mit mehr als 60 Gew.-% an gesättigten Fettsäuren, bezogen auf die gesamten Fettsäuren der pflanzlichen Butter mit hoher Sättigung, wobei die Gesamtmenge an pflanzlichen Butterarten mit hoher Sättigung wenigstens 50 Gew.-%, bezogen auf die erste pflanzliche Butterzusammensetzung, beträgt; und
(b) einen Farbstoff.

2. Feste kosmetische Zusammensetzung gemäß Anspruch 1, wobei der innere Kern 90-99 Gew.-% bildet und die äußere Schicht 1 bis 10 Gew.-% bildet, bezogen auf das kombinierte Gewicht des inneren Kerns und der äußeren Schicht.

3. Feste kosmetische Zusammensetzung gemäß Anspruch 1 oder 2, wobei der innere Kern pflanzliche Butterarten mit wenig Sättigung in einer Menge von 50 bis 80 Gew.-%, bezogen auf den inneren Kern, umfasst.

4. Feste kosmetische Zusammensetzung gemäß Anspruch 1, 2 oder 3, wobei der innere Kern Emulgator in einer Menge von 3 bis 20 Gew.-%, bezogen auf den inneren Kern, umfasst; und/oder wobei der Emulgator ausgewählt ist aus Polyoxyether von Laurylalkohol, PEG-6-Capryl/Capringlyceriden, PEG-60-Mandelglyceriden, Natriumcocoamphoacetat und Gemischen davon.

5. Feste kosmetische Zusammensetzung gemäß einem der Ansprüche 1 bis 4, wobei der innere Kern Farbstoff in einer Menge von 0,001 bis 5 Gew.-%, bezogen auf den inneren Kern, umfasst; und/oder wobei der Farbstoff in dem inneren Kern ein wasserlöslicher Farbstoff ist.

6. Feste kosmetische Zusammensetzung gemäß einem der Ansprüche 1 bis 5, wobei der innere Kern ferner pflanzliche Butterarten mit hoher Sättigung umfasst; wie z. B. der innere Kern pflanzliche Butterarten mit hoher Sättigung in einer Menge von 5 bis 25 Gew.-%, bezogen auf den inneren Kern, umfasst.

7. Feste kosmetische Zusammensetzung gemäß einem der Ansprüche 1 bis 6, wobei die äußere Schicht ferner pflanzliche Butterarten mit hoher Sättigung in einer Menge von wenigstens 60 Gew.-%, bezogen auf die äußere Schicht, umfasst; wie z. B. die äußere Schicht harte pflanzliche Butter in einer Menge von 80 bis 99,999 Gew.-%, bezogen auf die äußere Schicht, umfasst.

8. Feste kosmetische Zusammensetzung gemäß einem der Ansprüche 1 bis 7, wobei die äußere Schicht Farbstoff in einer Menge von 0,001 bis 5 Gew.-%, bezogen auf die äußere Schicht, umfasst; und/oder wobei der Farbstoff in der äußeren Schicht ein öllöslicher Farbstoff ist.

9. Feste kosmetische Zusammensetzung gemäß einem der Ansprüche 1 bis 8, wobei die äußere Schicht ferner pflanzliche Butterarten mit wenig Sättigung umfasst; wie z. B. die äußere Schicht pflanzliche Butterarten mit wenig Sättigung in einer Menge von 0,001 bis 10 Gew.-%, bezogen auf die äußere Schicht, umfasst.

10. Feste kosmetische Zusammensetzung gemäß einem der Ansprüche 1 bis 9, wobei die äußere Schicht ferner einen Emulgator umfasst.

11. Feste kosmetische Zusammensetzung gemäß Anspruch 1, umfassend
(i) einen inneren Kern, umfassend
(a) pflanzliche Butterarten mit wenig Sättigung in einer Menge von 50 bis 80 Gew.-%, bezogen auf den inneren Kern;
(b) einen Emulgator in einer Menge von 3 bis 20 Gew.-%, bezogen auf den inneren Kern; und
(c) einen Farbstoff in einer Menge von 0,001 bis 5 Gew.-%, bezogen auf den inneren Kern;
(ii) eine äußere Schicht, umfassend
(a) pflanzliche Butterarten mit hoher Sättigung in einer Menge von 80 bis 99,999 Gew.-%, bezogen auf die äußere Schicht;
(b) einen Farbstoff in einer Menge von 0,001 bis 5 Gew.-%, bezogen auf die äußere Schicht; und
(c) weiche pflanzliche Butter in einer Menge von 0 bis 10 Gew.-%, bezogen auf die äußere Schicht.

12. Feste kosmetische Zusammensetzung gemäß einem der Ansprüche 1 bis 11, wobei die pflanzlichen Butterarten mit hoher Sättigung ausgewählt sind aus Kakaobutter, Illipebutter, Murumurubutter, Kokumbutter und Gemischen davon.

13. Feste kosmetische Zusammensetzung gemäß einem der Ansprüche 1 bis 12, wobei die pflanzlichen Butterarten mit wenig Sättigung ausgewählt sind aus Aloebutter, Avocadobutter, Cupuacubutter, Makadamianussbutter, Mangobutter, Olivenbutter, Sheabutter, Kokosnussbutter, Kürbiskernbutter, Erdnussbutter, Mandelbutter, Kaffeebohnenbutter, raffinierter Butter, Hanfsamenbutter, Mochacchinobutter, Pistaziennussbutter, Shealoebutter und Gemischen davon.

14. Verfahren zur Herstellung einer festen kosmetischen Zusammensetzung gemäß Ansprüchen 1 bis 13, umfassend die Schritte:
i) Herstellen des inneren Kerns;
ii) Aufbringen der äußeren Schicht auf den inneren Kern.

15. Verfahren gemäß Anspruch 14, wobei die äußere Schicht auf den inneren Kern aufgebracht wird durch:
(a) Herstellen der Zusammensetzung der äußeren Schicht,
(b) Erhitzen der Zusammensetzung der äußeren Schicht auf eine Temperatur, bei der sie flüssig ist,
(c) Aufbringen der äußeren Schicht auf den inneren Kern durch Sprühen.

## Revendications

1. Composition cosmétique solide, comprenant
(i) un coeur interne qui est une deuxième composition de beurre végétal, où la deuxième composition de beurre végétal comprend
(a) un ou plusieurs beurres végétaux à faible saturation, où les un ou plusieurs beurres végétaux à faible saturation sont choisis parmi des beurres végétaux ayant moins de 60% en poids d'acides gras saturés sur la base des acides gras totaux du beurre végétal à faible saturation, où la quantité totale des beurres végétaux à faible saturation est d'au moins 50% en poids sur la base de la deuxième composition de beurre végétal ;
(b) un émulsifiant ; et
(c) un colorant ;
(ii) une couche externe qui est une première composition de beurre végétal, où la première composition de beurre végétal comprend
(a) un ou plusieurs beurres végétaux à saturation élevée, où les un ou plusieurs beurres végétaux à saturation élevée sont choisis parmi des beurres végétaux ayant plus de 60% en poids d'acides gras saturés sur la base des acides gras totaux du beurre végétal à saturation élevée, où la quantité totale des beurres végétaux à saturation élevée est d'au moins 50% en poids sur la base de la première composition de beurre végétal ; et
(b) un colorant.

2. Composition cosmétique solide selon la revendication 1, dans laquelle le coeur interne constitue 90-99% en poids et la couche externe constitue de 1 à 10% en poids, sur la base du poids combiné du coeur interne et de la couche externe.

3. Composition cosmétique solide selon la revendication 1 ou 2, dans laquelle le coeur interne comprend des beurres végétaux à faible saturation selon une quantité allant de 50 à 80% en poids, sur la base du coeur interne.

4. Composition cosmétique solide selon la revendication 1, 2 ou 3, dans laquelle le coeur interne comprend un émulsifiant selon une quantité allant de 3 à 20% en poids sur la base du coeur interne ; et/ou où l'émulsifiant est choisi parmi un polyoxyéther d'alcool laurylique, des glycérides capryliques/capriques de PEG-6, des glycérides d'amande de PEG-60, du cocoamphoacétate de sodium et des mélanges de ceux-ci.

5. Composition cosmétique solide selon l'une quelconque des revendications 1 à 4, dans laquelle le coeur interne comprend un colorant selon une quantité allant de 0,001 à 5% en poids sur la base du coeur interne ; et/ou où le colorant dans le coeur interne est un colorant hydrosoluble.

6. Composition cosmétique solide selon l'une quelconque des revendications 1 à 5, dans laquelle le coeur interne comprend en outre des beurres végétaux à saturation élevée ; tel que le coeur interne comprend des beurres végétaux à saturation élevée selon une quantité allant de 5 à 25% en poids sur la base du coeur interne.

7. Composition cosmétique solide selon l'une quelconque des revendications 1 à 6, dans laquelle la couche externe comprend des beurres végétaux à saturation élevée selon une quantité d'au moins 60% en poids sur la base de la couche externe ; tel que la couche externe comprend du beurre végétal dur selon une quantité allant de 80 à 99,999% en poids sur la base de la couche externe.

8. Composition cosmétique solide selon l'une quelconque des revendications 1 à 7, dans laquelle la couche externe comprend un colorant selon une quantité allant de 0,001 à 5% en poids sur la base de la couche externe ; et/ou où le colorant dans la couche externe est un colorant liposoluble.

9. Composition cosmétique solide selon l'une quelconque des revendications 1 à 8, dans laquelle la couche externe comprend en outre des beurres végétaux à faible saturation ; tel que la couche externe comprend des beurres végétaux à faible saturation selon une quantité allant de 0,001 à 10% en poids sur la base de la couche externe.

10. Composition cosmétique solide selon l'une quelconque des revendications 1 à 9, dans laquelle la couche externe comprend en outre un émulsifiant.

11. Composition cosmétique solide selon la revendication 1, comprenant
(i) un coeur interne comprenant
(a) des beurres végétaux à faible saturation, selon une quantité allant de 50 à 80% en poids sur la base du coeur interne ;
(b) un émulsifiant, selon une quantité allant de 3 à 20% en poids sur la base du coeur interne ; et
(c) un colorant, selon une quantité allant de 0,001 à 5% en poids sur la base du coeur interne ;
(ii) une couche externe comprenant
(a) des beurres végétaux à saturation élevée, selon une quantité allant de 80 à 99,999% en poids sur la base de la couche externe ;
(b) un colorant, selon une quantité allant de 0,001 à 5% en poids sur la base de la couche externe ; et
(c) un beurre végétal mou, selon une quantité allant de 0 à 10% en poids sur la base de la couche externe.

12. Composition cosmétique solide selon l'une quelconque des revendications 1 à 11, dans laquelle les beurres végétaux à saturation élevée sont choisis parmi le beurre de cacao, le beurre d'illipé, le beurre de murumuru, le beurre de kokum et des mélanges de ceux-ci.

13. Composition cosmétique solide selon l'une quelconque des revendications 1 à 12, dans laquelle les beurres végétaux à faible saturation sont choisis parmi le beurre d'aloe vera, le beurre d'avocat, le beurre de cupuaçu, le beurre de noix de Macadamia, le beurre de mangue, le beurre d'olive, le beurre de karité, le beurre de noix de coco, le beurre de graines de potiron, le beurre d'arachide, le beurre d'amande, le beurre de grains de café, le beurre raffiné, le beurre de graines de chanvre, le beurre de mochaccino, le beurre de pistache, le beurre de karité-aloe vera et des mélanges de ceux-ci.

14. Procédé de production d'une composition cosmétique solide telle que définie selon les revendications 1 à 13, comprenant les étapes
i) de préparation du coeur interne ;
ii) d'application de la couche externe au coeur interne.

15. Procédé selon la revendication 14, dans lequel la couche externe est appliquée au coeur interne par
(a) préparation de la composition de couche externe,
(b) chauffage de la composition de couche externe jusqu'à une température à laquelle elle est liquide,
(c) application de la couche externe au coeur interne par pulvérisation.
